# FASCICULE DE BREVET EUROPEEN

(11) **EP 3 202 322 B1**
(45) Date de publication et mention de la délivrance du brevet: **23.10.2024**
(21) Numéro de dépôt: 17154350.7
(22) Date de dépôt: 02.02.2017
(51) Int. Cl.: A61B 5/11, G08B 21/22, F21V 23/04, A47C 21/00, A61B 5/00

(54) **PROCEDE DE DETECTION DE PRESENCE ET/OU D'ANALYSE COMPORTEMENTALE A DISTANCE, SON DISPOSITIF DE MISE EN OEUVRE ET DISPOSITIF DE DETECTION DE PRESENCE**
VERFAHREN ZUR PRÄSENZDETEKTION UND/ODER ZUR VERHALTENSANALYSE PER FERNABFRAGE, VORRICHTUNG ZU DESSEN UMSETZUNG UND PRÄSENZDETEKTIONSVORRICHTUNG
METHOD OF DETECTING PRESENCE AND/OR REMOTE BEHAVIOURAL ANALYSIS, IMPLEMENTING DEVICE AND PRESENCE DETECTION DEVICE

(30) Priorité: 03.02.2016 FR 1650862
(43) Date de publication de la demande: 09.08.2017
(73) Titulaire: DOMALYS, 86240 Fontaine Le Comte (FR)
(72) Inventeur: BRILLAUD, Arnaud, 86240 Smarves (FR)
(74) Mandataire: Aquinov

(56) Documents cités:
- WO-A1-2013/014578
- WO-A1-2013/150187
- WO-A1-2015/036633
- WO-A1-2015/063644
- JP-A- 2004 133 500
- US-A1- 2012 314 901

## Description

La présente invention concerne un procédé de détection de présence et/ou d'analyse comportementale à distance, et son dispositif de mise en oeuvre ainsi qu'un dispositif de détection de présence spécifique, qui permettent de détecter à distance et de manière fiable, la chute d'une personne, et/ou une anomalie du comportement d'une personne au regard de ses habitudes de vie, et/ou la sortie d'un endroit d'une zone de l'habitation telle que la sortie d'un lit d'une personne.

L'invention sera plus particulièrement décrite, sans toutefois y être limitée, au regard de personnes âgées ou en perte d'autonomie, personnes hospitalisées, personnes à risque de chutes, etc.

Les personnes en perte d'autonomie ont tendance à se lever la nuit, pour aller aux toilettes par exemple, et à déambuler dans le noir. Dans ces circonstances, les risques de chutes sont importants.

Selon une première variante, un tapis est positionné au pied du lit des personnes en perte d'autonomie. Si la personne chute de son lit, le tapis amortit la chute. Ce dispositif n'est pas satisfaisant car le tapis n'empêche pas la chute et il peut être déplacé par inadvertance ce qui peut le rendre totalement inopérant s'il est malencontreusement placé sous le lit par exemple. Selon une deuxième variante, un radar de mouvement est fixé en hauteur sur un mur et transmet un signal dès qu'une personne bouge dans la pièce.

Ce mode de réalisation n'est pas satisfaisant car il est difficile de différencier un simple mouvement de la personne dans son lit d'une réelle sortie du lit (dans les deux cas, la personne bouge) et/ou de différencier une chute d'un retour au lit d'une personne (dans les deux cas la personne peut redevenir immobile).

De plus, les dispositifs existants sont dédiés à des sorties de lit, mais une chute peut arriver alors que la personne se déplace dans sa zone d'habitation. Or, il est primordial de pouvoir contacter un tiers, envoyer un message d'alerte en cas de chute. Cependant, les dispositifs d'alerte connus sont du type médaillon, bracelet ou boitier, tous, dotés d'un bouton sur lequel la personne doit appuyer pour prévenir de sa chute. Ces dispositifs ne sont pas complètement fiables, car la personne peut ne pas être en mesure d'appuyer sur le bouton, ne pas avoir sur elle le dispositif, peut avoir fait un malaise.

Le document WO2015/036633 A1 divulgue un autre exemple d'un dispositif de l'art antérieur.

La présente demande vise à remédier aux inconvénients de l'art antérieur en proposant un procédé de détection de présence et d'analyse comportementale à distance, et son dispositif de mise en oeuvre ainsi qu'un dispositif de détection spécifique, qui permettent de détecter à distance, de manière automatisée, et de manière fiable, la chute d'une personne, et/ou une anomalie du comportement d'une personne au regard de ses habitudes de vie, et/ou la sortie d'un endroit d'une zone de l'habitation telle que la sortie d'un lit d'une personne.

Selon un procédé utile à la compréhension de l'invention, le procédé de détection de la présence d'une personne dans une zone d'habitation et d'analyse comportementale à distance, comporte la fourniture de données de détection par des moyens de détection de présence lors de la détection de la personne dans au moins une zone de détection couverte par lesdits moyens de détection, la transmission des données par les moyens de détection à un module électronique d'acquisition de données, l'envoi à distance des données, depuis ledit module électronique d'acquisition via des moyens de communication (par exemple par wifi, radio, GSM...), à des moyens distants de traitement de données pour générer automatiquement au moins une information, et est caractérisé en ce que les moyens distants de traitement de données sont associés au moins une application logicielle qui est apte à générer automatiquement une ou plusieurs informations relatives à la chute de la personne depuis une station debout, assise ou couchée, en cas de chute effective de la personne dans la zone d'habitation, et/ou relatives au comportement de la personne dans la ou les zones de détection pour notamment en déduire une ou des anomalies de comportement.

Ainsi, le procédé permet de manière automatisée d'informer à distance un ou plusieurs tiers qui ne se trouvent pas dans la zone d'habitation, du comportement de la personne à se déplacer dans la zone d'habitation, de manière à détecter toute chute accidentelle et pouvoir intervenir rapidement, ou encore de manière très avantageuse, à analyser (éventuellement de manière statistique) ses déplacements (éventuellement par rapport aux déplacements habituels), pour prévenir d'une anomalie comportementale telle qu'un risque de chute prochaine, ou d'une pathologie.

On entend par « automatisée », la gestion des différents étapes du procédé par de seuls moyens techniques dont est doté le dispositif de mise en oeuvre, sans intervention humaine jusqu'à la transmission des informations de détection de chute et/ou d'analyse du comportement à des tiers distants de la personne.

Le procédé utile à la compréhension de l'invention est indépendant des facultés qu'auraient la personne surveillée à prévenir en cas de chute, appuyer sur bouton par exemple. Au contraire, le procédé de l'invention présente l'avantage de détecter et d'informer de manière automatisée sans intervention humaine, depuis la détection jusqu'à la réception de l'information par un tiers. Avantageusement, l'application logicielle est apte à fournir au moins les informations suivantes : l'entrée dans ou la sortie de la personne d'un endroit de la zone d'habitation telle que la sortie d'un lit, la sortie d'une pièce, le temps pour aller d'un endroit à un autre dans la zone d'habitation, et/ou le nombre de fois où la personne va d'un endroit à un autre de la zone d'habitation, et/ou le nombre de fois où la personne se lève à proximité de moyens de détection, et/ou le temps de présence de la personne en un endroit proche de moyens de détection et/ou la vitesse de déplacement de la personne.

A titre d'exemples nullement limitatifs :
- La vitesse de déplacement de la personne permet d'identifier que la personne se déplace extrêmement lentement ou bien plus lentement que d'habitude, et qu'un risque de chute existe.
- En détectant et analysant le nombre de fois où la personne va d'un endroit à un autre de la zone d'habitation, on identifie que la personne se lève bien plus souvent la nuit pour se rendre aux toilettes ; cette anomalie détectée peut correspondre notamment à une incontinence urinaire, avec le risque de chute associé du fait de la multiplicité des déplacements la nuit.
- Le comportement d'une personne qui passe bien moins de temps que d'habitude dans la cuisine, peut mettre en évidence un risque de déshydratation ou de mauvaise nutrition.
- La personne qui passe beaucoup de temps dans son lit ou dans une pièce sans se rendre dans sa cuisine, peut signifier un risque de pathologie.
- Peut être détectée l'intrusion dans l'habitation d'un intrus (cambrioleur, voleur), lorsque la personne est considérée comme absente (sortie de chez elle) ; la personne en quittant son habitation a alors informé de son départ les moyens de traitement des données à distance via un bouton/un système d'activation de chez elle ou via son smartphone par exemple, toute détection ultérieure de présence dans l'habitation génère alors un message d'alarme.
- Peut être détectée l'introduction dans la maison d'un intrus (cambrioleur, voleur) alors que la personne est chez elle seule ; en disposant de plusieurs modules de détection de présence dans les pièces de l'habitation et en ayant activé l'application informatique d'intrusion, les moyens de traitement des données permettent de combiner les informations émises par les différents modules de détection et d'en déduire/vérifier la présence d'un individu non désiré, et d'envoyer un message d'alarme.

Le procédé utile à la compréhension de l'invention comprend une étape d'éclairage automatisé dans la zone de détection et/ou dans un autre endroit (tel que les pièces suivantes pour allumer de manière préventive les autres pièces à proximité), en réponse à la réception par le module électronique d'acquisition, de données de détection de présence transmises par les moyens de détection, en particulier l'éclairage étant activé si la luminosité dans la zone de détection est inférieure à une valeur seuil. De plus, l'intensité lumineuse de l'éclairage peut être progressivement augmentée.

Selon une autre caractéristique, le procédé met en oeuvre en tant que moyens de détection de présence au moins deux capteurs de position dans un plan vertical et décalés en hauteur l'un par rapport à l'autre (capteurs dits capteur haut et capteur bas), en particulier les capteurs étant agencés de sorte que leur secteur angulaire de détection considéré dans un plan vertical ne se coupe pas dans la zone de détection, le module électronique de traitement des données étant apte à détecter la chute d'une personne en fonction des données transmises par les capteurs, les données du module électronique relatives à la détection de chute étant destinées à être transmises aux moyens distants de traitement des données.

La séquence d'acquisition et traitement des données par le module électronique de détection pour détecter une chute est la suivante : détection de présence par le capteur haut, puis analyse de détection du capteur bas, vérification qu'il n'y a pas de détection de présence par le capteur haut pendant un temps donné (ce qui correspond à identifier que la personne a chuté, est au sol et qu'elle ne s'est pas relevée), et confirmation qu'il y a une détection de présence par le capteur bas validant la présence de la personne au sol et donc sa chute.

Selon une autre caractéristique, le procédé comporte, en fonction de la ou des informations générées par les moyens de traitement des données et l'application logicielle, une ou des étapes d'affichage desdites informations et/ou d'envoi de messages. Les informations peuvent se présenter sous diverses formes, texte, graphiques, courbes, chiffres, etc.. Les messages sont par exemple, des SMS, MMS, notifications, messages vocaux, sonneries, etc... Ainsi, à titre d'exemple, tous tiers, la famille, un professionnel tel qu'un aide-soignant, peut recevoir des alertes sur son téléphone portable, son smartphone, de manière à intervenir immédiatement en cas de chute détectée, ou à contacter un docteur pour vérifier si une pathologie n'est pas en cause en cas d'anomalie comportementale détectée, ou prévenir les premiers signes d'une dégradation de santé.

L'application logicielle peut notamment adresser chaque jour à un ou plusieurs tiers intéressés un message que l'activité/le comportement de la personne surveillée est normale, et en cas de comportement anormal, délivre instantanément une notification d'alerte.

Au regard de la détection d'une chute, la séquence d'acquisition et traitement des données par le module électronique de détection pour détecter la chute est la suivante : détection de présence par le capteur haut, puis analyse de détection du capteur bas, vérification qu'il n'y a pas de détection de présence par le capteur haut pendant un temps donné (ce qui correspond à identifier que la personne a chuté, est au sol et qu'elle ne s'est pas relevée), et confirmation qu'il y a une détection de présence par le capteur bas validant la présence de la personne au sol et donc sa chute. Une fois que le module électronique a détecté la chute, les données d'analyse de chute sont transmises aux moyens distants de traitement des données qui engendreront un ou plusieurs envois d'informations, dont en particulier des messages d'alerte.

A noter que le capteur haut est positionné à une hauteur telle qu'un animal de compagnie ou un enfant n'est pas pris en compte par le capteur haut et n'entraine pas de fausses alertes.

L'invention peut également être résumée par un dispositif selon la revendication 1 et une procédé selon la revendication 9, avec des réalisations préférées selon les revendications dépendantes.

Des modes de réalisation avantageux sont décrits dans les revendications dépendantes.

D'autres caractéristiques et avantages ressortiront de la description qui va suivre de l'invention, description donnée à titre d'exemple uniquement, en regard des dessins annexés sur lesquels :
- La figure 1 illustre schématiquement le procédé de détection de présence et d'analyse comportementale d'une personne dans une zone d'habitation, selon l'invention ;
- La figure 2 est une représentation schématique en vue de dessus d'une zone d'habitation tel qu'un appartement, comprenant plusieurs pièces dans lesquelles sont disposés des dispositifs détection de présence selon l'invention ;
- La figure 3 est une représentation schématique en vue de dessus d'une pièce équipée d'un dispositif de détection selon une première variante de l'invention ;
- La figure 4 est une vue latérale du lit équipé du dispositif de détection de la figure 3 ;
- La figure 5 est une représentation schématique d'un module de détection configuré pour communiquer avec un poste de contrôle selon une première variante de l'invention ;
- Les figures 6A et 6B sont des vues qui illustrent un premier mode de fonctionnement de l'invention ;
- Les figures 7A et 7B sont des vues qui illustrent un deuxième mode de fonctionnement de l'invention ;
- Les figures 8A à 8C sont des vues latérales d'une pièce équipée d'un dispositif de détection selon une deuxième variante de l'invention
- La figure 9 est une vue en perspective d'un dispositif/module de détection de présence selon un mode de réalisation de l'invention ;
- Les figures 10A et 10B sont des vues schématiques en coupe verticale des zones de détection d'un module de détection doté de deux capteurs décalés en hauteur.

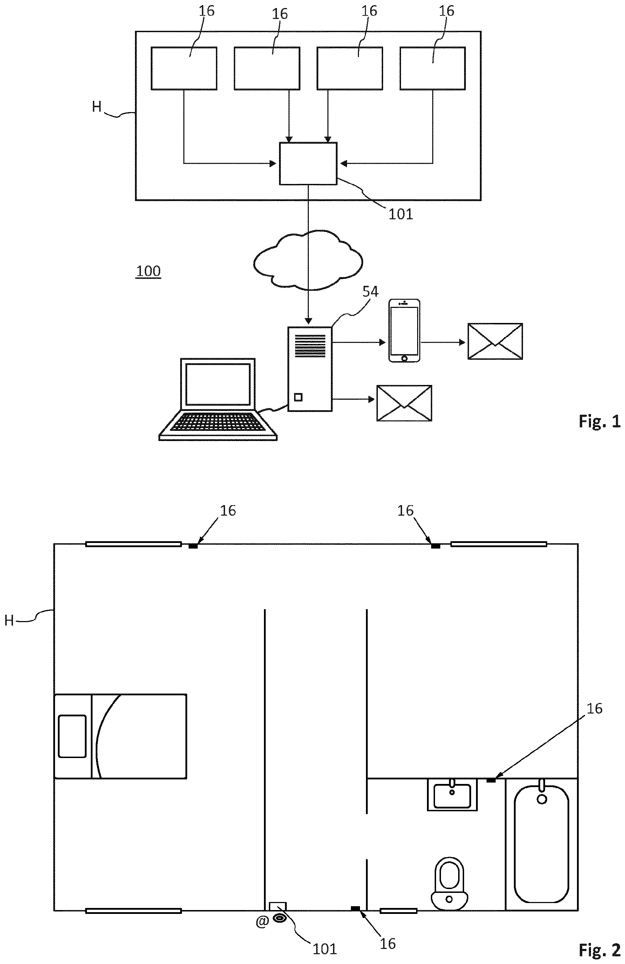

La figure 1 illustre schématiquement le procédé selon l'invention de détection de présence et d'analyse comportementale à distance d'une personne dans une zone d'habitation H et son dispositif de mise en oeuvre 100.

Le procédé de détection de présence et d'analyse comportementale à distance selon l'invention consiste à détecter la chute d'une personne dans son environnement d'habitation, les risques de chutes, et les anomalies comportementales correspondant à des premiers signes d'une dégradation ou permettant de mettre en évidence des pathologies naissantes, et cela de manière fiable et automatisée, sans intervention humaine jusqu'à la transmission des informations de détection de chute et/ou d'analyse du comportement à des tiers distants de la personne. En fonction des résultats transmis, le ou les tiers habilités pourront intervenir. Le dispositif de mise en oeuvre de détection et d'analyse comportementale 100 comporte selon l'invention :
- Dans l'habitation H, plusieurs dispositifs de détection de présence 16 (ou dits encore dans la description « modules de détection ») qui sont répartis dans différentes pièces de l'habitation, chaque dispositif/module de détection étant apte à détecter une personne dans la zone de détection couverte par ledit module de détection et à transmettre à distance les données de détection de présence ;
- Une interface de communication à distance 101 pour transmettre les données depuis les modules de détection 16 vers l'extérieur de l'habitation H à des moyens distants de traitement de données 54;
- En dehors et à distance de l'habitation H, les moyens distants de traitement de données 54 (nommé encore plus loin « plateforme ») qui sont associés à au moins une application logicielle pour traiter les données reçues et transmises par les modules de détection 16, et générer automatiquement une ou plusieurs informations relatives à la chute d'une personne depuis une station debout, assise ou couchée, en cas de chute effective de la personne dans la zone d'habitation, et/ou relatives au comportement d'une personne dans la ou les zones de détection pour notamment en déduire une ou des anomalies de comportement.

Un module de détection 16 comporte des moyens de détection de présence tels que des capteurs, des moyens électroniques d'acquisition de données recevant les données fournies par les capteurs, lesdits moyens étant en outre aptes à transmettre les données à distance jusqu'à l'interface de communication 101. L'envoi des données dans l'habitation jusqu'à l'interface de communication 101 peut se faire par voie filaire, ou de préférence par ondes radio, par exemple par wifi ou Bluetooth.

Des exemples de réalisation de module de détection sont donnés plus loin.

L'envoi des données depuis l'interface de communication 101 vers l'extérieur de l'habitation peut être filaire ou non selon l'emplacement des moyens distants de traitement de données 54. En particulier, si lesdits moyens de traitement 54 sont dans le même bâtiment que l'habitation H, la liaison de communication peut se faire par un réseau Ethernet ou CPL. La communication se fera sinon par Internet.

L'application logicielle permet d'horodater et d'analyser les données pour notamment fournir au moins les informations suivantes : l'entrée dans ou la sortie de la personne d'un endroit de la zone d'habitation telle que la sortie d'un lit, d'une pièce, le temps pour aller d'un endroit à un autre dans la zone d'habitation, et/ou le nombre de fois où la personne va d'un endroit à un autre de la zone d'habitation, et/ou le nombre de fois où la personne se lève à proximité de moyens de détection, et/ou le temps de présence de la personne en un endroit proche de moyens de détection.

De plus, les moyens de traitement des données et l'application logicielle sont aptes à fournir les informations par affichage sur des écrans de visualisation du type écran d'ordinateur, tablette, smartphone, et/ou par le renvoi de messages vers des appareils de réception du type téléphone, smartphone, bipeur... Les messages se présentent par exemple sous la forme de SMS, MMS, notification, signaux sonores et/ou de texte.

Selon le type d'informations, ces dernières seront présentées sous la forme de textes, chiffres, graphes, etc...

En fonction des informations engendrées et messages reçus, et cela de manière automatisée depuis la détection de présence des déplacements de la personne dans l'habitation, le ou les tiers recevant les informations et/ou messages peuvent alors intervenir directement ou indirectement en prévenant les personnes ad hoc.

La figure 2 illustre une installation dotée de plusieurs modules de détection 16, chacun agencé dans une pièce distincte de l'habitation, par exemple, la chambre, le couloir, la salle de bain et la pièce de vie. Chacun des modules de détection 16 est apte à détecter les déplacements dans sa zone de détection et à transmettre à l'interface commune de communication 101, les données de détection.

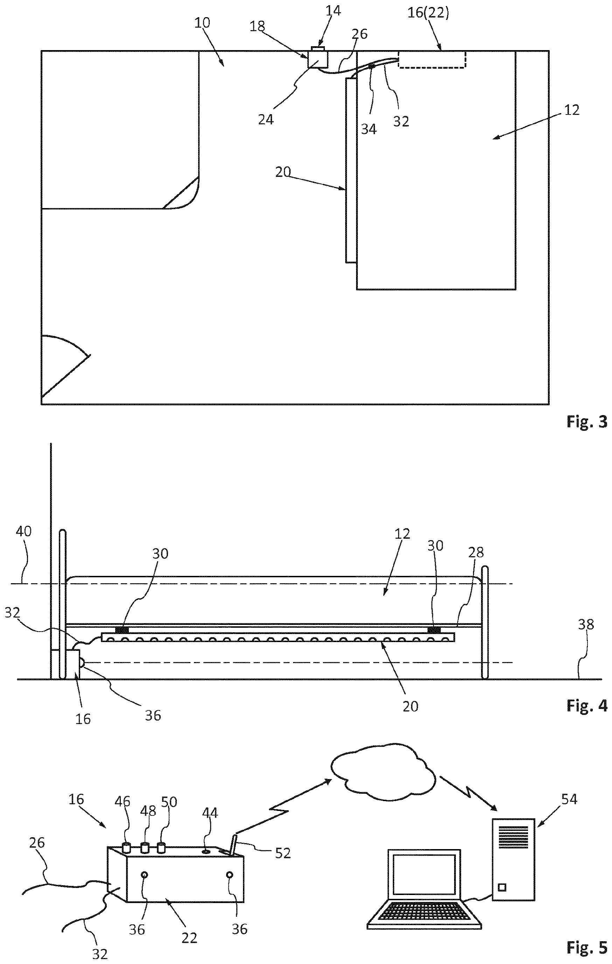

Sur la figure 3, on a représenté une pièce 10 comprenant au moins un lit 12 ainsi qu'au moins une prise électrique 14.

Selon un mode de réalisation, le dispositif de détection comprend un module de détection 16, un module d'alimentation 18 et au moins un système d'éclairage 20.

Selon une première variante, le module de détection 16 se présente sous la forme d'un boitier 22 disposé au sol, sous le lit 12, au niveau de la tête du lit 12. Dans cette position, le module de détection 16 ne peut pas être déplacé par inadvertance. Le module de détection 16 étant positionné au niveau de la tête du lit 12, un seul module de détection 16 suffit pour surveiller tout le pourtour du lit 12.

De préférence, le module d'alimentation 18 est un boitier 24 distinct du boitier 22 du module de détection 16. Dans ce cas, les deux modules 16 et 18 sont reliés par une liaison filaire 26. Cette solution permet de positionner idéalement le module de détection 16 quelle que soit la position de la prise électrique 14. En variante, le module de détection 16 et le module d'alimentation 18 sont regroupés dans un unique boitier.

Selon un mode de réalisation, le module d'alimentation 18 comprend une fiche mâle apte à se brancher sur la prise électrique 14 positionnée sur une première face, au moins une prise électrique positionnée sur une autre face et une connexion pour relier la liaison filaire 26.

Selon un mode de réalisation, le système d'éclairage 20 se présente sous la forme d'un élément longiligne qui comprend une pluralité de diodes électroluminescentes (DEL) réparties sur sa longueur ainsi qu'une liaison configurée pour relier de façon démontable sans outil le système d'éclairage 20 au lit 12.

Selon une configuration, le lit 12 comprend un châssis métallique 28 avec un longeron horizontal. Dans ce cas, la liaison comprend au moins un aimant 30 permettant de relier de manière démontable, sans avoir besoin d'outil, le système d'éclairage 20 au lit 12. Bien entendu, l'invention n'est pas limitée à ce mode de réalisation pour le système d'éclairage 20 ou la liaison.

Selon un mode de réalisation, le module de détection 16 et le système d'éclairage 20 sont distincts et reliés par une liaison filaire 32. De préférence, cette liaison filaire 32 comprend un premier tronçon relié au module de détection 16, un second tronçon relié au système d'éclairage 20 et une connectique 34 pour relier les premier et second tronçons. Avantageusement, le dispositif de détection comprend un moyen de détection d'un mauvais branchement de la connectique 34 ainsi qu'un moyen pour générer une alerte lorsque le moyen de détection détermine que la connectique 34 n'est pas correctement branchée et que par conséquent, le système d'éclairage 20 n'est pas opérationnel.

L'alerte peut être un signal lumineux et/ou un signal sonore et/ou un message.

En variante, le module de détection 16 et le système d'éclairage 20 sont intégrés dans un seul et même élément.

Le module de détection 16 comprend au moins un capteur de détection directif 36 configuré pour détecter la présence d'une personne dans une zone de détection située entre le sol 38 et le plan supérieur 40 du lit 12. Ainsi, ce capteur 36 permet de différencier les mouvements d'une personne sur le lit des mouvements d'une personne à l'extérieur du lit 12.

Pour la présente invention, le plan supérieur 40 du lit est un plan horizontal positionné approximativement au niveau ou très légèrement en dessous de la face supérieure du matelas du lit.

Sur une première variante visible sur les figures 2 et 3 par exemple, le module de détection 16 comprend un unique capteur de détection directif 36 positionné à proximité du sol 38 pour détecter la présence d'une personne dans une zone de détection située sous le plan supérieur 40 du lit. Selon cette variante, le module de détection directif 16 peut comprendre deux capteurs positionnés à proximité du sol, orientés selon deux directions différentes.

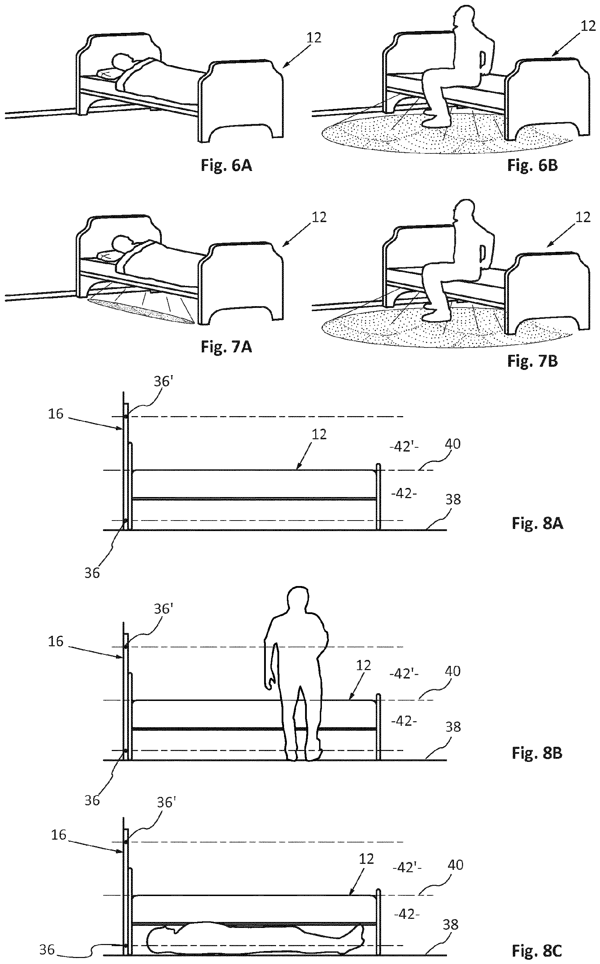

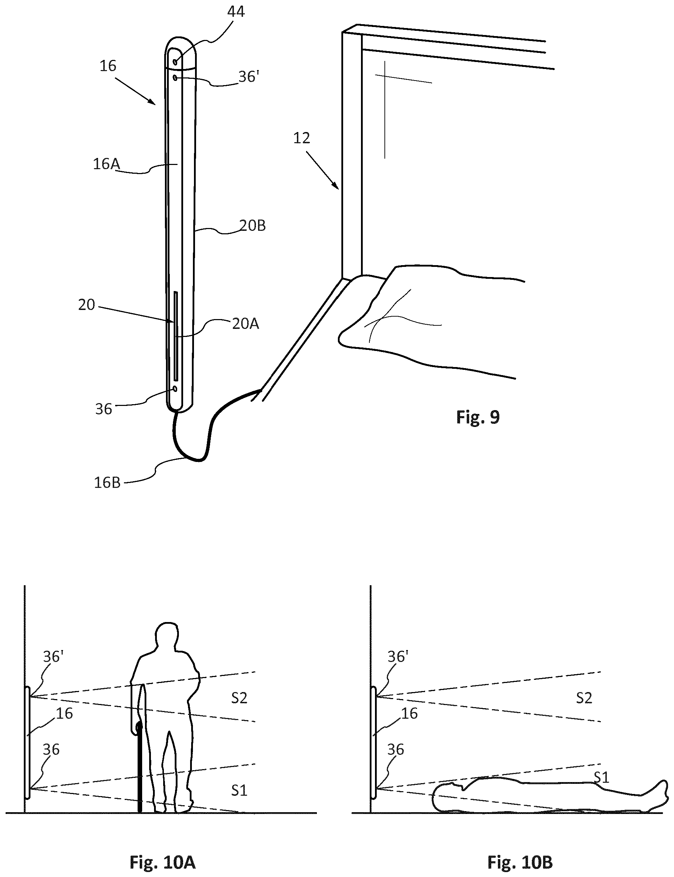

Selon une deuxième variante visible sur les figures 8A à 8C, et 9, le module de détection 16 comprend deux capteurs de détection directifs 36 et 36', un premier capteur de détection directif 36 positionné à proximité du sol 38 pour détecter la présence d'une personne dans une première zone de détection 42 située sous le plan supérieur 40 du lit et un second capteur directif 36' positionné au-dessus du plan supérieur 40 du lit pour détecter la présence d'une personne dans une seconde zone de détection 42' située au-dessus du plan supérieur 40 du lit. Selon cette deuxième variante, le module de détection 16 comprend au moins deux capteurs de détection directifs décalés en hauteur.

Cette deuxième variante permet de différencier les chutes d'un simple déplacement dans la pièce 10. En effet, en cas de chute, seul le premier capteur 36 détecte la personne. A contrario, lorsqu'une personne se déplace dans la pièce, les deux capteurs 36 et 36' détectent la personne.

Quelle que soit la variante, le capteur de détection directif 36 positionné à proximité du sol 38 est positionné dans un plan horizontal sous le lit 12. Par conséquent, il ne peut pas détecter les mouvements de la personne sur le lit et ne détecte que les mouvements de sortie du lit. Selon un mode de réalisation, chaque capteur de détection directif 36, 36' est un capteur de type Infra-rouge passif (PIR) directif configuré pour détecter la présence d'une personne uniquement dans une zone de détection délimitée par deux plans horizontaux. A titre indicatif, un capteur peut couvrir un secteur angulaire de 90° à 110° dans un plan horizontal et un secteur angulaire de 80° dans un plan vertical. De préférence, en cas de détection pour une chute, le secteur angulaire dans un plan vertical sera plutôt de 30°.

Bien entendu, l'invention n'est pas limitée à ce type de capteur.

Selon une autre caractéristique, le module de détection 16 comprend un capteur de luminosité 44.

Selon un mode de réalisation visible sur la figure 4, le module de détection 16 comprend une première commande 46 pour activer ou non un mode d'envoi de message et/ou une deuxième commande 48 pour activer ou non un mode veilleuse et/ou une troisième commande 50 pour régler une temporisation.

Selon une autre caractéristique, le module de détection 16 comprend un moyen de contrôle, comme une électronique par exemple, configuré pour gérer l'activation ou non du système d'éclairage 20, pour ajuster l'intensité de l'éclairage, pour gérer l'envoi ou non d'un ou plusieurs messages ainsi qu'un système de communication 52 configuré pour communiquer avec une plateforme 54.

Lorsque plusieurs dispositifs de détection communiquent avec la même plateforme 54, chaque dispositif de détection comprend un identifiant qui lui est propre. L'identifiant du dispositif de détection 16 émettant le message est incorporé dans le message pour permettre à la plateforme 54 d'identifier le dispositif de détection 16 ayant émis le message.

La plateforme 54 comprend au moins un ordinateur ainsi qu'un logiciel configuré pour lister les messages reçus provenant d'au moins un module de détection 16, les horodater, réaliser un renvoi d'au moins un message vers un téléphone d'une personne en fonction du paramétrage.

En variante, les dispositifs de détection peuvent transmettre des messages à un système d'alerte prévu généralement dans les établissements d'hébergement pour personnes âgées dépendantes.

Selon une autre variante, le module de détection comprend une entrée pour connecter une commande actionnable par la personne alitée, comme par exemple une commande appel malade.

Le dispositif de détection précédemment décrit est polyvalent et peut fonctionner selon différents modes de fonctionnement.

Lorsque la luminosité détectée par le capteur de luminosité 44 est supérieure à un seuil donnée, le dispositif de détection 16 est à l'état passif. Par conséquent, la détection d'un mouvement par le ou les capteur(s) de détection directif(s) 36 n'engendre pas l'activation du système d'éclairage 20 et/ou l'envoi d'un message.

Lorsque la luminosité détectée par le capteur de luminosité 44 est inférieure au seuil donné, le dispositif de détection 16 est à l'état actif.

Si le mode veilleuse n'est pas activé, lorsque le ou les capteur(s) de détection directif(s) 36 ne détecte(nt) aucun mouvement, le système d'éclairage 20 n'émet pas de lumière, comme illustré sur la figure 6A.

Dès qu'un capteur de détection directif 36 détecte un mouvement, le module de détection 16 commande l'activation du système d'éclairage 20 qui émet alors une lumière comme illustré sur la figure 6B. De préférence, le dispositif de détection comprend une régulation configurée pour augmenter progressivement l'intensité lumineuse émise par le système d'éclairage 20 au moment de son activation.

Si le mode veilleuse est activé grâce à la commande 48, le système d'éclairage 20 fonctionne dès que le dispositif de détection 16 est à l'état actif. En l'absence de mouvement, le système d'éclairage 20 émet une lumière avec une faible intensité qui correspond par exemple à 5% de sa valeur maximale, comme illustré sur la figure 7A. Dès qu'un capteur de détection directif 36 détecte un mouvement, le module de détection 16 commande l'activation du système d'éclairage 20 qui émet alors une lumière comme illustré sur la figure 7B. Comme indiqué précédemment, la régulation permet d'augmenter progressivement l'intensité lumineuse émise par le système d'éclairage 20 au moment de son activation.

Indépendamment du mode veilleuse, lorsque le système d'éclairage 20 a été activé, il reste activé tant qu'un mouvement est détecté par le ou les capteur(s) de détection directif(s) 36. Lorsqu'aucun mouvement n'est détecté pendant une durée donnée, le module de détection 16 commande la désactivation du système d'éclairage 20 qui s'éteint lorsque le mode veilleuse n'est pas activé ou revient à une faible intensité d'éclairage lorsque le mode veilleuse est activé.

La durée donnée peut être régulée grâce à la commande 50.

Lorsque le mode envoi de message est activé grâce à la commande 46, le module de détection 16 transmet un message à chaque activation du système d'éclairage 20. Ce message est transmis à une plateforme 54 qui effectue une traçabilité des activations des systèmes d'éclairage 20 des différents dispositifs de détection. En fonction du paramétrage, la plateforme 54 peut réaliser un renvoi d'appel vers un téléphone d'un surveillant ou vers un poste de surveillance. A la réception d'un message, le surveillant sait qu'une personne est sortie du lit et peut alors décider des actions à entreprendre. En présence de plusieurs dispositifs de détection reliés à une même plateforme 54, le message transmis au surveillant ou au poste de surveillance comprend un identifiant permettant d'identifier la personne qui est sortie du lit.

Lorsque le module de détection 16 comprend plusieurs capteurs décalés en hauteur, le module de détection 16 peut différencier les messages transmis en fonction de la détection d'un mouvement par les deux capteurs ou uniquement par celui positionné en bas.

Ainsi, le module de détection 16 transmet un premier type de message lorsque seul le premier capteur de détection directif 36 positionné à proximité du sol détecte la présence d'une personne. Le même module de détection 16 ne transmet aucun message ou transmet un deuxième type de message lorsque les deux capteurs de détection directifs 36 et 36' détectent la présence d'une personne.

Plus particulièrement, la séquence d'acquisition et traitement des données par le module électronique de détection pour détecter une chute est la suivante : détection de présence par le capteur haut, puis analyse de détection du capteur bas, vérification qu'il n'y a pas de détection de présence par le capteur haut pendant un temps donné (ce qui correspond à identifier que la personne a chuté, est au sol et qu'elle ne s'est pas relevée), et confirmation qu'il y a une détection de présence par le capteur bas validant la présence de la personne au sol et donc sa chute.

La figure 9 montre un exemple de réalisation d'un dispositif/module de détection 16, agencé à proximité d'un lit 12.

Le module de détection 16 présente la forme d'une colonne monobloc comprenant dans un corps longiligne 16a, au moins deux capteurs 36 et 36' disposés en façade du corps et aux deux extrémités distales respectives du corps, dits capteurs haut et bas, et un système d'éclairage 20.

De préférence, le système d'éclairage comporte un éclairage direct 20A agencé en façade, et un rétro-éclairage 20B éclairant depuis l'arrière et les côtés du corps.

Avantageusement, le système d'éclairage 20 se déclenche automatiquement dès que les capteurs détecte une présence, et le degré d'éclairement peut être régulé en fonction de la luminosité.

Les moyens d'acquisition de données des capteurs de détection de présence 36 et 36' et les commande et régulation du système d'éclairage 20 en fonction de la détection de présence et de la luminosité, sont intégrés à l'intérieur du corps 16A du module, de manière non visible depuis l'extérieur.

Certains paramètres liés à l'analyse de présence pourront être réglés ou activés depuis des boutons situés sur le corps du module et/ou à partir de l'application logicielle via une interface d'affichage et d'interaction du type smartphone ou autre.

Enfin, l'ensemble des éléments nécessitant une alimentation électrique sont reliés à l'intérieur du corps à un alimentation commune qui est connectée à un fil d'alimentation extérieur 16B, lui-même adapté pour se brancher sur une prise électrique.

Le module monobloc représenté en figure 9 permet avantageusement de rassembler l'ensemble des moyens techniques à la détection de présence et à l'éclairage, et de fournir pour l'utilisateur un module aisé de mise en oeuvre, prêt à son branchement et son fonctionnement, du type « Plug and Play » en anglais.

Tel qu'illustré schématiquement sur les figures 10A et 10B, le module de détection 16 est placé verticalement, à distance du sol, par exemple de l'ordre de 10 à 20 cm du sol, les deux capteurs bas 36 et haut 36' étant distants l'un de l'autre de l'ordre, entre 70 et 110 cm.

Les deux capteurs bas 36 et haut 36', de préférence du type par infrarouge, sont conçus pour délivrer des ondes de détection de manière directive et selon un secteur angulaire limité dans le plan vertical, respectivement S1 et S2, afin que le secteur angulaire de l'un ne rencontre pas le secteur angulaire de l'autre. Ainsi, le capteur haut détectera la partie basse d'une personne, tandis que le capteur haut détectera la partie supérieure d'une personne. En cas de chute (figure 10B), les secteurs angulaires ne se recouvrant pas, les moyens de traitement 54 associés à l'application logicielle analyseront systématiquement de manière fiable un chute lorsque le capteur bas détectera une présence, tandis que le capteur haut ne détectera aucune présence.

## Revendications

1. Dispositif de détection de présence d'une personne dans une zone de détection, pour détecter une chute de personne, une entrée ou sortie d'une zone de détection telle qu'une sortie de lit, une sortie d'une pièce, comportant au moins deux capteurs de détection directifs (36, 36'), positionnés dans un plan vertical et décalés en hauteur l'un par rapport à l'autre, dans lequel le dispositif comporte au moins un système d'éclairage (20) et au moins un capteur de luminosité (44), le système d'éclairage étant activé en fonction de la luminosité et de la détection d'une présence, l'éclairage étant activé si la luminosité dans la zone de détection est inférieure à une valeur seuil et lorsque l'un des capteurs de détection directifs (36, 36') détecte un mouvement et dans lequel le dispositif présente un corps de forme longiligne, à la manière d'une colonne, comprenant au moins en façade les deux au moins capteurs distants de détection de présence (36, 36') qui sont disposés à proximité des deux extrémités distales respectives du corps, et intégrant ledit système d'éclairage (20).

2. Dispositif selon la revendication 1, **caractérisé en ce que** le premier capteur de détection directif (36) est positionné à proximité du sol, tandis que le second capteur directif (36') est positionné au-dessus d'un plan correspondant sensiblement à la hauteur du bassin d'une personne se tenant debout ou à la hauteur des épaules d'une personne assise, ou au-dessus du plan supérieur d'un lit.

3. Dispositif selon la revendication 1, **caractérisé en ce que** les deux capteurs directifs sont agencés de sorte que leur secteur angulaire de détection considéré dans un plan vertical ne se coupe pas dans la zone de détection.

4. Dispositif selon l'une quelconque des revendications précédentes, **caractérisé en ce qu'**il se présente sous la forme d'un module à corps monobloc qui comporte les capteurs de présence (36, 36') associés à des moyens d'acquisition de données des capteurs, et les commande et régulation du système d'éclairage (20), de préférence les éléments du module nécessitant une alimentation électrique étant reliés à l'intérieur du corps du module à une alimentation commune connectée à un fil d'alimentation extérieur (16B), en particulier le module étant du type « Plug and Play ».

5. Dispositif selon l'une quelconque des revendications précédentes, **caractérisé en ce qu'**il comporte un module de détection et d'acquisition des données (16), qui est associé aux capteurs de présence (36, 36') et reçoit les données des capteurs, un système de communication (52) configuré pour communiquer à distance avec des moyens de traitement des données (54) qui sont associés à au moins une application logicielle configurée pour analyser les données transmises par le module de détection et d'acquisition, horodater les données, et engendrer au moins une information de résultat d'analyse, de préférence en réalisant un renvoi d'au moins un message.

6. Dispositif selon l'une quelconque des revendications précédentes, **caractérisé en ce qu'**il comporte au moins une caméra.

7. Dispositif selon l'une quelconque des revendications précédentes, **caractérisé en ce qu'**il comporte d'autres éléments fonctionnels de détection, tels que des capteurs de température, d'humidité, de monoxyde de carbone, de fumée, lesdits éléments fonctionnels de détection étant aptes à communiquer avec des moyens de traitement des données qui sont associés à une application logicielle.

8. Installation comportant plusieurs dispositifs de détection de présence selon l'une quelconque des revendications précédentes, et éventuellement comportant d'autres éléments fonctionnels de détection, tels que des capteurs de température, d'humidité, de monoxyde de carbone, de fumée, **caractérisée en ce que** les dispositifs de détection de présence ou autres éléments fonctionnels de détection sont positionnés à différents endroits d'une zone d'habitation tels que à proximité d'un lit, dans un endroit de passage de la zone d'habitation, dans un couloir, une salle de bain, les toilettes, une cuisine, une pièce de vie, une chambre, une pièce de séjour, une cuisine, en particulier l'ensemble des dispositifs de détection de présence et éléments fonctionnels de détection étant aptes à communiquer avec des moyens communs de traitement des données associés à une application logicielle commune, de manière à rassembler et analyser l'ensemble des données des différents dispositifs de la zone d'habitation, notamment les données transmises incorporent l'identifiant du dispositif de détection, la date et l'heure, l'identifiant de chaque capteur et la valeur de chaque capteur.

9. Procédé de détection de présence utilisant au moins un dispositif de détection de présence selon l'une quelconque des revendications 1 à 7 dans au moins une zone de détection, dans lequel le procédé comprend une étape d'éclairage automatisé dans la zone de détection et/ou dans un autre endroit, en réponse à la détection de mouvement détecté par ledit dispositif.

10. Procédé de détection de présence selon la revendication précédente, caractérisé en qu'il comporte en outre un étape de traitement des données acquises par le dispositif pour générer automatiquement une ou plusieurs informations relatives à la chute d'une personne depuis une station debout, assise ou couchée, en cas de chute effective de la personne dans la zone d'habitation, et/ou relatives au comportement d'une personne dans la ou les zones de détection pour notamment en déduire une ou des anomalies de comportement.

11. Procédé de détection de présence selon la revendication précédente, caractérisé en que l'étape de traitement des données pour générer lesdites informations est réalisée par des moyens de traitement de données distants dudit au moins un dispositif, lesdits moyens de traitement étant associés à au moins une application logicielle.

12. Procédé de détection de présence selon la revendication 10 ou 11, caractérisé en que lesdites informations portent sur : l'entrée dans ou la sortie de la personne d'un endroit de la zone d'habitation telle que la sortie d'un lit, la sortie d'une pièce, le temps pour aller d'un endroit à un autre dans la zone d'habitation, et/ou le nombre de fois où la personne va d'un endroit à un autre de la zone d'habitation, et/ou le nombre de fois où la personne se lève à proximité de moyens de détection, et/ou le temps de présence de la personne en un endroit proche de moyens de détection.

13. Procédé selon la revendication 10 ou 11, **caractérisé en ce qu'**il comporte, en fonction de la ou des informations générées par les moyens de traitement des données et l'application logicielle, une ou des étapes d'affichage desdites informations et/ou d'envoi de messages.

## Patentansprüche

1. Vorrichtung zum Erfassen einer Anwesenheit einer Person in einem Erfassungsbereich zum Erfassen eines Sturzes der Person, eines Betretens oder Verlassens eines Erfassungsbereichs, wie ein Verlassen eines Bettes, ein Verlassen eines Raums, die mindestens zwei richtungsabhängige Erfassungssensoren (36, 36') aufweist, die in einer vertikalen Ebene und höhenversetzt zueinander positioniert sind, wobei die Vorrichtung mindestens ein Beleuchtungssystem (20) und mindestens einen Helligkeitssensor (44) aufweist, wobei das Beleuchtungssystem in Abhängigkeit von der Helligkeit und der Erfassung einer Anwesenheit aktiviert wird, wobei die Beleuchtung aktiviert wird, falls die Helligkeit in dem Erfassungsbereich kleiner als ein Schwellenwert ist und wenn der eine der richtungsabhängigen Erfassungssensoren (36, 36') eine Bewegung erfasst, und wobei die Vorrichtung einen säulenartigen länglichen Körper vorweist, der mindestens auf der Vorderseite die mindestens zwei entfernten Anwesenheitserfassungssensoren (36, 36') umfasst, die in der Nähe von zwei jeweiligen distalen Enden des Körpers angeordnet sind, und das Beleuchtungssystem (20) integriert.

2. Vorrichtung nach Anspruch 1, **dadurch gekennzeichnet, dass** der erste richtungsabhängige Erfassungssensor (36) in der Nähe des Bodens positioniert ist, wohingegen der zweite richtungsabhängige Sensor (36') über einer Ebene, die im Wesentlichen der Höhe des Beckens einer aufrechtstehenden Person oder der Höhe der Schultern einer sitzenden Person entspricht, oder über der oberen Ebene eines Bettes positioniert ist.

3. Vorrichtung nach Anspruch 1, **dadurch gekennzeichnet, dass** die zwei richtungsabhängigen Sensoren so eingerichtet sind, dass sich ihr Winkelerfassungsabschnitt, der in einer vertikalen Ebene betrachtet wird, in dem Erfassungsbereich nicht schneidet.

4. Vorrichtung nach einem der vorstehenden Ansprüche,
**dadurch gekennzeichnet, dass** sie in der Form eines Moduls mit einem einteiligen Körper vorliegt, der die Anwesenheitssensoren (36, 36'), die mit Mitteln zum Aufnehmen von Daten der Sensoren verknüpft sind, und die Steuerung und die Regelung des Beleuchtungssystems (20) aufweist, wobei vorzugsweise die Elemente des Moduls, die eine Stromzufuhr benötigen, im Inneren des Körpers des Moduls an eine gemeinsame Zufuhr angeschlossen sind, die mit einem externen Zuführungsdraht (16B) verbunden ist, insbesondere wobei das Modul von der Art "Plug and Play" ist.

5. Vorrichtung nach einem der vorstehenden Ansprüche,
**dadurch gekennzeichnet, dass** sie ein Erfassungs- und Datenaufnahmemodul (16) aufweist, das mit den Anwesenheitssensoren (36, 36') verknüpft ist und die Daten von den Sensoren empfängt, ein Kommunikationssystem (52), das für eine Fernkommunikation mit Datenverarbeitungsmitteln (54) konfiguriert ist, die mit mindestens einer Softwareanwendung verknüpft sind, die zum Analysieren von Daten, die durch das Erfassungs- und Aufnahmemodul übertragen werden, Zeitstempeln der Daten und Erzeugen mindestens einer Analyseergebnisinformation konfiguriert ist, vorzugsweise indem ein erneutes Senden mindestens einer Nachricht ausgeführt wird.

6. Vorrichtung nach einem der vorstehenden Ansprüche,
**dadurch gekennzeichnet, dass** sie mindestens eine Kamera aufweist.

7. Vorrichtung nach einem der vorstehenden Ansprüche,
**dadurch gekennzeichnet, dass** sie andere funktionale Erfassungselemente wie Temperatur-, Feuchtigkeits-, Kohlenmonoxid- und Rauchsensoren aufweist, wobei die funktionalen Erfassungselemente geeignet sind, um mit Datenverarbeitungsmitteln zu kommunizieren, die mit einer Softwareanwendung verknüpft sind.

8. Anlage, die mehrere Anwesenheitserfassungsvorrichtungen nach einem der vorstehenden Ansprüche und optional weitere funktionale Erfassungselemente wie Temperatur-, Feuchtigkeits-, Kohlenmonoxid- und Rauchsensoren aufweist, **dadurch gekennzeichnet, dass** die Anwesenheitserfassungsvorrichtungen oder die anderen funktionalen Erfassungselemente an unterschiedlichen Orten eines Wohnbereichs positioniert sind, wie in der Nähe eines Bettes, in einem Durchgangsort des Wohnbereichs, in einem Flur, einem Bad, der Toilette, einer Küche, einem Wohnraum, einem Schlafzimmer, einem Aufenthaltsraum, einer Küche, wobei insbesondere die Gesamtheit der Anwesenheitserfassungsvorrichtungen und der funktionalen Erfassungselemente geeignet ist, um mit gemeinsamen Datenverarbeitungsmitteln zu kommunizieren, die mit einer gemeinsamen Softwareanwendung verknüpft sind, um die Gesamtheit der Daten der unterschiedlichen Vorrichtungen des Wohnbereichs zusammenzutragen und zu analysieren, wobei besonders die übertragenen Daten die Kennung der Erfassungsvorrichtung, das Datum und die Uhrzeit, die Kennung jedes Sensors und den Wert jedes Sensors beinhalten.

9. Anwesenheitserfassungsverfahren unter Verwendung mindestens einer Anwesenheitserfassungsvorrichtung nach einem der Ansprüche 1 bis 7 in mindestens einem Erfassungsbereich, wobei das Verfahren einen Schritt zum automatisierten Beleuchten in dem Erfassungsbereich und/oder an einem anderen Ort als Reaktion auf die Erfassung der Bewegung umfasst, die durch die Vorrichtung erfasst wird.

10. Anwesenheitserfassungsverfahren nach dem vorstehenden Anspruch, **dadurch gekennzeichnet, dass** es ferner einen Schritt zum Verarbeiten der Daten, die durch die Vorrichtung aufgenommen werden, zum automatischen Generieren einer oder mehrerer Informationen über den Sturz einer Person aus einer stehenden, sitzenden oder liegenden Stellung im Falle eines tatsächlichen Sturzes der Person in dem Wohnbereich und/oder über das Verhalten einer Person in dem oder den Erfassungsbereich(en) zum besonders daraus Ableiten einer oder mehrerer Verhaltensanomalien umfasst.

11. Anwesenheitserfassungsverfahren nach dem vorstehenden Anspruch, **dadurch gekennzeichnet, dass** der Datenverarbeitungsschritt zum Generieren der Informationen durch Datenverarbeitungsmittel ausgeführt wird, die von der mindestens einen Vorrichtung entfernt sind, wobei die Verarbeitungsmittel mit mindestens einer Softwareanwendung verknüpft sind.

12. Anwesenheitserfassungsverfahren nach Anspruch 10 oder 11,
**dadurch gekennzeichnet, dass** sich die Informationen beziehen auf: das Betreten oder das Verlassen der Person eines Ortes des Wohnbereichs, wie das Verlassen eines Bettes, das Verlassen eines Raums, die Zeit zum Gehen von einem Ort zu dem anderen in dem Wohnbereich, und/oder wie oft die Person von einem Ort zu dem anderen im Wohnbereich geht, und/oder wie oft die Person in der Nähe des Erfassungsmittels aufsteht, und/oder die Anwesenheitszeit der Person an einem Ort nahe des Erfassungsmittels.

13. Verfahren nach Anspruch 10 oder 11, **dadurch gekennzeichnet, dass** es in Abhängigkeit von der oder den Information(en), die durch die Datenverarbeitungsmittel und die Softwareanwendung generiert wird/werden, einen oder mehrere Schritte zum Anzeigen der Informationen und/oder zum Senden von Nachrichten aufweist.

## Claims

1. A device for detecting the presence of a person in a detection zone, for detecting a person's fall, an entry into or exit from a detection zone such as getting out of bed, leaving a room, comprising at least two directional detection sensors (36, 36'), positioned in a vertical plane and offset in height in relation to one another, wherein the device comprises at least one lighting system (20) and at least one luminosity sensor (44), the lighting system being activated based on the luminosity and the detection of a presence, the lighting being activated if the luminosity in the detection zone is less than a threshold value and when one of the directional detection sensors (36, 36') detects movement, and wherein the device has a slender, column-like body, comprising at least on the front the at least two remote presence detection sensors (36, 36'), which are arranged proximate to the two respective distal ends of the body, and integrating said lighting system (20).

2. The device according to claim 1, **characterized in that** the first directional detection sensor (36) is positioned proximate to the floor, while the second directional sensor (36') is positioned above a plane that corresponds substantially to the height of the pelvis of a standing person or to the height of the shoulders of a seated person, or above the upper plane of a bed.

3. The device according to claim 1, **characterized in that** the two directional sensors are arranged so that their angular detection sector considered in a vertical plane does not intersect the detection zone.

4. The device according to any one of the preceding claims,
**characterized in that** it is in the form of a module with a one-piece body which comprises the presence sensors (36, 36') associated with sensor data acquisition means, and the control and regulation of the lighting system (20), preferably the elements of the module requiring a power supply being connected inside the body of the module to a common power supply connected to an external power cord (16B), in particular the module being of the "Plug and Play" type.

5. The device according to any one of the preceding claims,
**characterized in that** it comprises a data detection and acquisition module (16),
which is associated with the presence sensors (36, 36') and receives the data from the sensors, a communication system (52) configured to communicate remotely with data processing means (54) which are associated with at least one software application configured to analyze the data transmitted by the detection and acquisition module, time-stamp the data, and generate at least one analysis result information item, preferably by returning at least one message.

6. The device according to any one of the preceding claims,
**characterized in that** it comprises at least one camera.

7. The device according to any one of the preceding claims,
**characterized in that** it comprises other functional detection elements, such as sensors of temperature, humidity, carbon monoxide, smoke, said functional detection elements being able to communicate with data processing means associated with a software application.

8. An installation comprising several presence detection devices according to any one of the preceding claims, and optionally comprising other functional detection elements, such as sensors of temperature, humidity, carbon monoxide, smoke sensors, **characterized in that** the presence detection devices or other functional detection elements are positioned at different locations in a living area, such as near a bed, in a hallway of the living area, in a corridor, a bathroom, a powder room, a kitchen, a living room, a bedroom, a den, a kitchen, in particular all the presence detection devices and functional detection elements being able to communicate with common data processing means associated with a common software application, so as to gather and analyze all the data from the various devices in the living area, in particular the transmitted data incorporating the identifier of the detection device, the date and time, the identifier of each sensor and the value of each sensor.

9. A presence detection method using at least one presence detection device according to any one of claims 1 to 7 in at least one detection zone, wherein the method comprises a step of automated lighting in the detection zone and/or in another location, in response to the detection of movement detected by said device.

10. The presence detection method according to the preceding claim, **characterized in that** it further comprises a step for processing the data acquired by the device in order to automatically generate one or more items of information relating to a person's fall from a standing, sitting or lying position, in the event of the person actually falling in the living area, and/or relating to the behavior of a person in the detection zone(s) in order, in particular, to deduce one or more behavioral anomalies therefrom.

11. The presence detection method according to the preceding claim, **characterized in that** the data processing step for generating said information is performed by data processing means remote from said at least one device, said processing means being associated with at least one software application.

12. The presence detection method according to claim 10 or 11, **characterized in that** said information relates to: the person's entry into or exit from a location in the living area, such as getting out of bed, leaving a room, the time taken to go from one place to another in the living area, and/or the number of times the person goes from one place to another in the living area, and/or the number of times the person stands up in the vicinity of detection means, and/or the time the person is present in a place close to detection means.

13. The method according to claim 10 or 11, **characterized in that** it comprises, based on the information item(s) generated by the data processing means and the software application, one or more steps for displaying said information and/or sending messages.
